(19) Europäisches Patentamt
European Patent Office
Office européen des brevets

(11) EP 0 994 854 B1

# (12) FASCICULE DE BREVET EUROPEEN

(45) Date de publication et mention de la délivrance du brevet:
**18.09.2002 Bulletin 2002/38**

(21) Numéro de dépôt: **98935072.3**

(22) Date de dépôt: **01.07.1998**

(51) Int Cl.$^7$: **C07D 209/44**

(86) Numéro de dépôt international:
**PCT/FR98/01405**

(87) Numéro de publication internationale:
**WO 99/001430 (14.01.1999 Gazette 1999/02)**

## (54) PROCEDE DE PREPARATION D'UN PERHYDROISOINDOLE SUBSTITUE

VERFAHREN ZUR HERSTELLUNG EINES SUBSTITUIERTES PERHYDROISOINDOLS

METHOD FOR PREPARING A SUBSTITUTED PERHYDROISOINDOLE

(84) Etats contractants désignés:
**AT BE CH CY DE DK ES FI FR GB GR IE IT LI LU MC NL PT SE**

(30) Priorité: **03.07.1997 FR 9708431**

(43) Date de publication de la demande:
**26.04.2000 Bulletin 2000/17**

(73) Titulaire: **ADIR**
**F-92415 Courbevoie Cedex (FR)**

(72) Inventeurs:
- **LECOUVE, Jean-Pierre**
  **F-76600 Le Havre (FR)**
- **FUGIER, Claude**
  **F-76600 Le Havre (FR)**
- **SOUVIE, Jean-Claude**
  **F-76210 Bolbec (FR)**

(56) Documents cités:
**EP-A- 0 507 534**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un procédé de synthèse industriel d'un perhydroisoindole substitué de formule (I) :

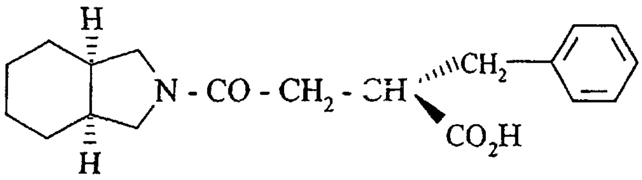

(I)

et de ses sels pharmaceutiquement acceptables.

Le composé de formule (I) ainsi que ses sels d'addition possèdent des propriétés pharmacologiques particulièrement intéressantes. C'est un insulinosécréteur très puissant, ce qui le rend utile dans le traitement des diabètes insulino-dépendants.

Le composé de formule (I), sa préparation et son utilisation en thérapeutique ont été décrits dans le brevet européen EP 0 507 534. Toutefois, la préparation industrielle d'un dérivé tel que le dérivé de formule (I) nécessite une étude approfondie de toutes les étapes réactionnelles, du choix des matières premières, des réactifs et des solvants permettant d'obtenir les rendements optima.

Le procédé de synthèse du composé de formule (I) décrit dans le brevet EP 0 507 534 ne permet pas d'obtenir ce composé avec un rendement optimum. En effet, l'obtention de l'isomère intéressant par la voie de synthèse décrite ne permet pas d'accéder à la régiosélectivité souhaitée. Il est alors nécessaire de réaliser de nombreuses purifications pour obtenir cet isomère de "qualité pharmaceutique".

Compte-tenu de l'intérêt pharmaceutique de ce composé et de l'absence d'un procédé permettant son obtention avec un bon rendement, une pureté satisfaisante et, si possible, à partir de matières premières peu onéreuses et disponibles dans le commerce, des recherches plus approfondies ont été entreprises et ont abouti à la mise au point d'un nouveau procédé de synthèse particulièrement intéressant.

L'invention a plus précisément pour objet un procédé de préparation du composé de formule (I) caractérisé en ce que l'on fait réagir le diméthylsuccinate avec le benzaldéhyde en milieu méthanolique.
pour conduire au diacide de formule (II) :

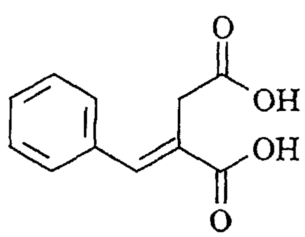

(II)

qui, après chauffage dans un solvant aprotique tel que le tétrahydrofurane ou l'éther isopropylique en présence d'anhydride acétique, conduit à l'anhydride de formule (III):

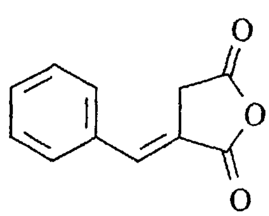

(III)

que l'on fait réagir avec le perhydroisoindole de formule (IV) dans un solvant aprotique tel que le toluène, l'acétonitrile, l'acétate d'éthyle, le méthyltertiobutyléther ou le tétrahydrofurane, ou encore dans un mélange tétrahydrofurane/toluène,

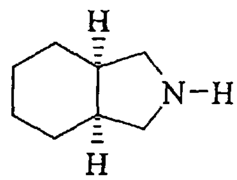

(IV)

pour conduire au composé de formule (V) :

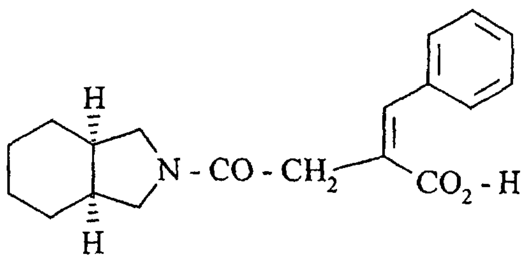

(V)

qui subit une hydrogénation catalytique en utilisant comme catalyseur d'hydrogénation asymétrique le complexe Rhodium / (2S.4S)-N-butoxycarbonyl-4-diphénylphosphino-2-diphénylphosphinométhylpyrrolidine ou Rh / (S,S) BPPM, en milieu méthanolique ou chlorométhylénique, suivie d'une salification en présence d'une amine A pour conduire au composé de formule (VI) :

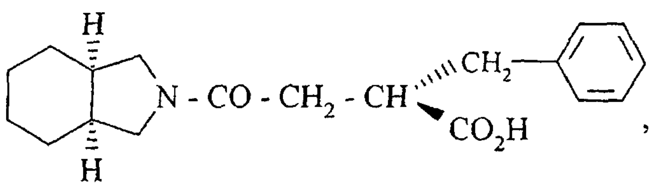

, A (VI)

qui :

en milieu basique en présence d'un sel minéral, conduit à un sel d'addition du composé de formule (I) que l'on transforme, si on le souhaite, en acide correspondant,

ou, en milieu acide, conduit au composé de formule (I), que l'on transforme, si on le souhaite, en sel d'addition pharmaceutiquement acceptable.

Parmi les sels d'addition pharmaceutiquement acceptables, on peut citer, à titre non limitatif, les sels de sodium, de calcium sous forme hydratée ou non.

Le sel d'addition préféré est le sel de calcium.

Ce procédé est particulièrement intéressant pour les raisons suivantes :

- L'ouverture de l'anhydride de formule (III) par le perhydroisoindole de formule (IV) permet d'obtenir une très grande régiosélectivité. En effet, le composé de formule (V) est isolé avec une régiosélectivité supérieure à 99,5 %.
- La réduction énantiosélective du composé de formule (V) par le complexe Rh/(S,S)BPPM fournit un excès énantiomérique supérieur à 92 %. Le rapport molaire complexe/substrat utilisé dans cette étape est de 1/2000 à 1/20000 et de préférence de 1/2000 à 1/10000.

Le complexe de Rhodium Rh/(S,S)BPPM est connu comme catalyseur d'hydrogénation énantiosélective. Cependant, dans le méthanol ou le chlorure de méthylène, solvants de réaction, l'acide de formule (I/a) a tendance à évoluer vers un mélange de régioisomères (I/a)/(I/b) :

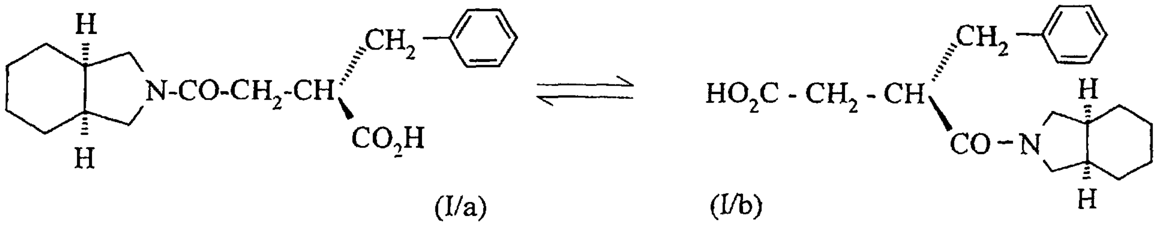

(I/a) (I/b)

Une étude cinétique a permis de montrer que le pourcentage de régioisomère (I/b) augmente rapidement en fonction du temps et de la température. Or, il a été montré que lorsque le composé (I) est en présence d'une amine A, la formation du composé de formule (I/b) est considérablement ralentie.

A titre d'exemple, à 65°C après 12 heures en solution dans le méthanol, le pourcentage de composé (I/b) est d'environ 9 % lorsque le composé est sous forme d'acide libre alors que celui-ci est d'environ 1 % lorsque le composé est sous forme de sel d'une amine A. Ceci représente un avantage considérable dans la mise au point de ce procédé industriel.

Le sel de calcium est obtenu à partir de ces sels organiques par addition d'hydroxide de sodium dans une proportion de 0,4 à 1 équivalent suivie par l'addition de chlorure de calcium dans une proportion de 1 à 1,3 équivalents.

Le sel de calcium peut aussi être obtenu par addition d'ammoniaque dans une proportion de 0,3 à 1 équivalent suivie par l'addition de chlorure de calcium dans une proportion de 1 à 1,3 équivalents. Ces réactions de salification sont effectuées en milieu aqueux ou hydroalcoolique tel qu'un mélange éthanol/eau pouvant contenir jusqu'à 96 % d'éthanol.

D'autre part, la cristallisation du sel obtenu est facilement applicable au niveau industriel et permet une excellente purification énantiomérique et chimique du produit attendu.

Elle permet d'autre part d'éliminer toute trace de catalyseur.

Parmi les amines A utilisables dans cette étape de réaction, on peut citer la (R)-1-phényléthylamine, la morpholine, la N-méthylmorpholine ou la cyclohexylamine. Les amines préférées sont la (R)-1-phényléthylamine et la morpholine.

Les exemples suivants illustrent l'invention mais ne la limitent en aucune façon.

***EXEMPLE 1* : Dihydrate de bis-2-(S)-benzyl-4-oxo-4-(cis-perhydroisoindol-2-yl)butyrate de calcium**

*Stade A : Acide benzylidène succinique*

A 290 mmoles de méthylate de sodium dissoutes dans 80 ml de méthanol, on ajoute 700 mmoles de diméthylsuccinate puis 20 ml de méthanol. Le milieu est porté au reflux et 236 mmoles de benzaldéhyde sont ajoutées lentement puis 20 ml de méthanol. L'ensemble est maintenu sous agitation une heure au reflux et 100 ml de méthanol sont alors distillés. Au milieu réactionnel concentré, on ajoute 120 ml d'eau et 120 ml de soude 10N. La distillation du méthanol est poursuivie. Le résidu est dilué par 150 ml d'eau. Après addition de dichlorométhane, le diacide précipite par addition lente d'acide chlorhydrique 12N. Le diacide est filtré, lavé par du dichlorométhane puis de l'eau. Après séchage, on obtient le produit attendu.

Point de fusion : 199°C

*Stade B : Anhydrique benzylidène succinique*

291 mmoles du composé obtenu au stade précédent sont mis en suspension dans 180 ml d'éther isopropylique. 320 mmoles d'anhydride acétique sont ajoutées et la suspension portée au reflux 3 h 30. Après refroidissement à 4°C, filtration de l'anhydride formée, lavage par de l'éther isopropylique, on obtient le produit attendu.

Point de fusion : 168°C

*Stade C : Acide 2-[(cis-perhydroisoindol-2-yl)carbonylméthyl]-3-phénylacrylique*

A 167 mmoles de l'anhydride obtenu au stade précédent en suspension dans 250 ml de toluène est ajoutée très lentement une solution de 175 mmoles de perhydroisoindoline dans 32 ml de toluène. L'ensemble est refroidi à -5°C. Le précipité de monoamide obtenu est filtré et lavé par du toluène glacé. Après séchage, on obtient le produit attendu.

Point de fusion : 162°C

*Stade D : 2-(S)-Benzyl-4-oxo-4-(cis-perhydroisoindol-2-yl)-butyrate de 1-(R)-phényléthylamine*

80 mmoles de l'amide obtenue au stade précédent sont dissoutes dans 75 ml de méthanol. 40 micromoles de (S,S)BPPM sont dissoutes dans 15 ml de méthanol, 20 micromoles de $[Rh(COD)Cl]_2$ dans 15 ml de méthanol. Les solutions sont dégazées, chargées dans le réacteur d'hydrogénation puis hydrogénées à 20°C sous 5 bars. A la solution méthanolique d'hydrogénation, on ajoute 250 ml de toluène et à 5°C une solution de 82,5 mmoles de (R)1-phényléthylamine dans 100 ml de toluène. Le méthanol est chassé sous pression réduite à température ambiante et on ajoute 300 ml de toluène. Le précipité formé est filtré à 20°C et lavé par deux fois 20 ml de toluène. Après séchage, le sel brut obtenu est recristallisé dans de l'acétone, filtré et séché, et conduit au produit attendu .
Point de fusion : 144°C

*Stade E : Dihydrate de bis-2-(S)-benzyl-4-oxo-4-(cis-perhydroisoindol-2-yl)butyrate de calcium*

A 9 mmoles du sel de 1-(R)-phényléthylamine précédemment purifié dissoutes dans 80 ml d'une solution aqueuse d'ammoniaque à 1.8 %, on ajoute 13 ml d'une solution aqueuse contenant 9 mmoles de chlorure de calcium dihydrate. Le précipité obtenu est filtré, lavé à l'eau et séché, et conduit au produit attendu.
Point de fusion : 214°C
$[\alpha]^{20}_{365}$ = + 32,4 (c = 5%, MeOH)

***EXEMPLE 2 : Dihydrate de bis-2-(S)-benzyl-4-oxo-4-(cis-perhydroisoindol-2-yl)butyrate de calcium***

*Stades A, B et C : Ces stades sont identiques aux stades A, B et C de l'exemple 1.*

*Stade D : 2-(S)-Benzyl-4-oxo-4-(cis-perhydroisoindol-2-yl)-butyrate de morpholine*

L'hydrogénation est effectuée comme précédemment. A la solution méthanolique d'hydrogénation glacée, on ajoute 69 g de morpholine et concentre sous vide à une température inférieure à 25°C pour éliminer le méthanol. On ajuste le concentrat à une masse de 115 g par ajout de morpholine, puis ajoute 250 ml de méthyltertiobutyléther et agite pendant 20 h à l'ambiante.
Le sel d'amine précipité est filtré et lavé par un mélange méthyltertiobutyléther-morpholine puis par du méthyltertiobutyléther. Après séchage, on obtient le produit attendu.
Point de fusion : 110°C

*Stade E : Dihydrate de bis-2-(S)-benzyl-4-oxo-4-(cis-perhydroisoindol-2-yl)butyrate de calcium*

A 25 mmoles du sel de morpholine précédemment purifié dissoutes dans 75 ml d'éthanol et 30 ml d'eau, on ajoute 25 ml de soude 1N puis 28 ml d'une solution aqueuse contenant 12,5 mmoles de chlorure de calcium dihydrate. Le précipité obtenu est filtré, lavé et séché, et conduit au produit attendu.
Point de fusion : 214°C
$[\alpha]\frac{20}{365}$ = + 32,4 (c = 5%, MeOH)

## Revendications

**1.** Procédé de préparation industrielle d'un perhydroisoindole substitué de formule (I) :

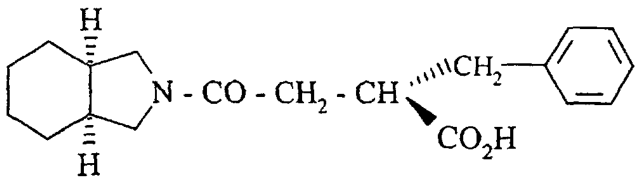

(I)

et de ses sels pharmaceutiquement acceptables, **caractérisé en ce que** l'on fait réagir le diméthylsuccinate avec le benzaldéhyde en milieu méthanolique,
pour conduire au diacide de formule (II):

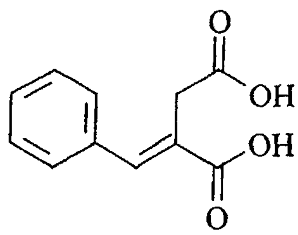

(II)

qui, après chauffage en milieu aprotique en présence d'anhydride acétique, conduit à l'anhydride de formule (III) :

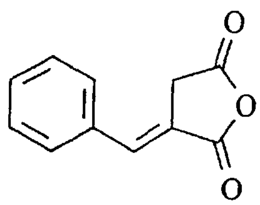

(III)

que l'on fait réagir avec la perhydroisoindoline de formule (IV) dans un solvant aprotique

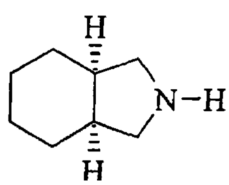

(IV)

pour conduire au composé de formule (V) :

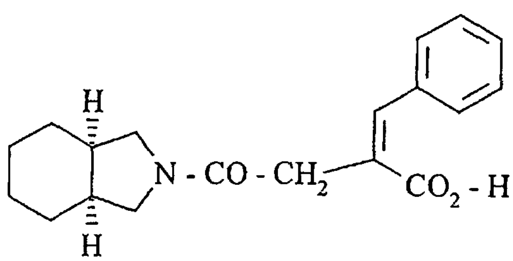

(V)

qui subit une hydrogénation catalytique en utilisant comme catalyseur d'hydrogénation asymétrique le complexe Rhodium / (2S,4S)-N-butoxycarbonyl-4-diphénylphosphino-2-diphénylphosphinométhylpyrrolidine ou Rh / (S,S) BPPM, en milieu méthanolique ou chlorométhylénique, suivie d'une salification en présence d'une amine A pour conduire au composé de formule (VI):

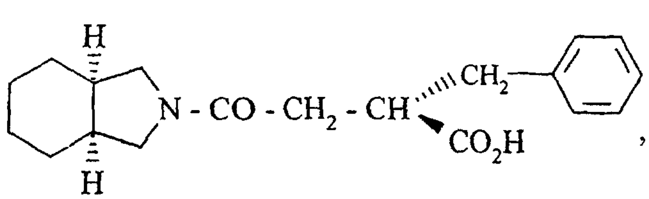

, A (VI)

qui :

en milieu basique en présence d'un sel minéral, conduit à un sel d'addition du composé de formule (I) que l'on transforme, si on le souhaite, en acide correspondant,
ou, en milieu acide, conduit au composé de formule (I), que l'on transforme, si on le souhaite, en sel d'addition pharmaceutiquement acceptable.

**2.** Procédé de préparation selon la revendication 1 **caractérisé en ce que** l'amine A utilisée est la (R)-1-phényléthylamine.

**3.** Procédé de préparation selon la revendication 1 **caractérisé en ce que** l'amine A est la morpholine.

**4.** Procédé de préparation selon la revendication 1 **caractérisé en ce que** l'amine A est la N-méthylmorpholine.

**5.** Procédé de préparation selon la revendication 1 **caractérisé en ce que** l'amine A est la cyclohexylamine.

**6.** Procédé de préparation selon la revendication 1 du sel de calcium de l'acide bis-2-(S)-benzyl-4-oxo-4-(cis-perhydroisoindol-2-yl)butyrique.

**7.** Procédé de préparation selon la revendication 1 du dihydrate de bis-2-(S)-benzyl-4-oxo-4-(cis-perhydroisoindol-2-yl)butyrate de calcium.

## Claims

**1.** Process for the industrial preparation of a substituted perhydroisoindole of formula (I):

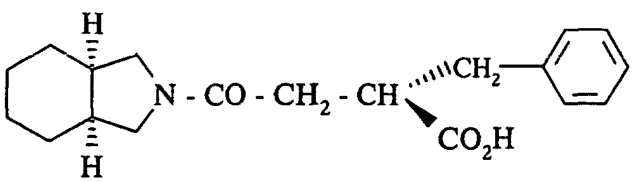

(I)

and of pharmaceutically acceptable salts thereof, **characterised in that** dimethyl succinate is reacted with benzaldehyde in methanolic medium,
to yield the diacid of formula (II):

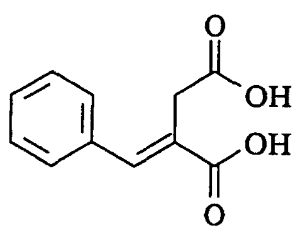

(II)

which, after heating in an aprotic medium in the presence of acetic anhydride, yields the anhydride of formula (III) :

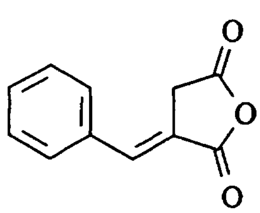

(III)

which is reacted with the perhydroisoindoline of formula (IV) in an aprotic solvent

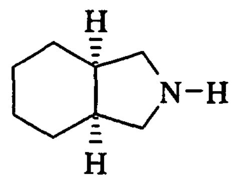

(IV)

to yield the compound of formula (V):

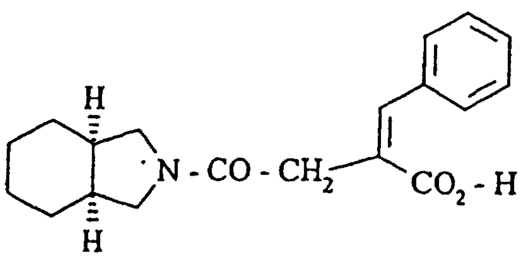

(V)

which is subjected to catalytic hydrogenation using as asymmetric hydrogenation catalyst the complex rhodium / (2S,4S)-N-butoxycarbonyl-4-diphenylphosphino-2-diphenylphosphinomethylpyrrolidine or Rh / (S,S)-BPPM, in methanolic or methylene chloride medium, followed by conversion to a salt in the presence of an amine A to yield a compound of formula (VI):

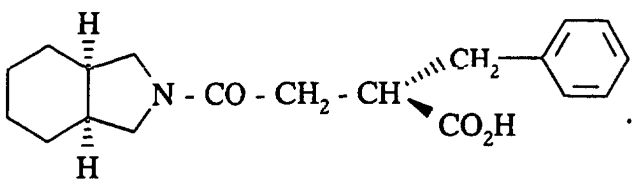

A (VI)

which:

in a basic medium in the presence of a mineral salt yields an addition salt of the compound of formula (I), which is converted, if desired, into the corresponding acid, or,
in an acidic medium yields the compound of formula (I), which is converted, if desired, into a pharmaceutically acceptable addition salt.

**2.** Preparation process according to claim 1, **characterised in that** the amine A used is (R)-1-phenylethylamine.

**3.** Preparation process according to claim 1, **characterised in that** the amine A is morpholine.

**4.** Preparation process according to claim 1, **characterised in that** the amine A is N-methyl-morpholine.

**5.** Preparation process according to claim 1, **characterised in that** the amine A is cyclohexylamine.

**6.** Process according to claim 1 for the preparation of the calcium salt of bis-2-(S)-benzyl-4-oxo-4-(cis-perhydroisoindol-2-yl)butyric acid.

**7.** Process according to claim 1 for the preparation of calcium bis-2-(S)-benzyl-4-oxo-4-(cis-perhydroisoindol-2-yl) butyrate dihydrate.

## Patentansprüche

1. Verfahren zur technischen Herstellung eines substituierten Perhydroisoindols der Formel (I):

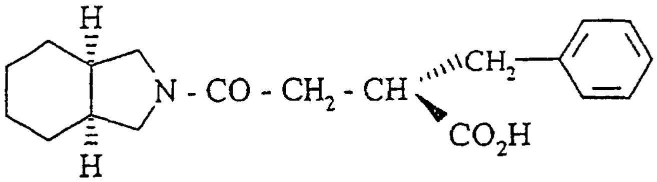

(I)

und seiner pharmazeutisch annehmbaren Salze, **dadurch gekennzeichnet, daß** man Dimethylsuccinat mit Benzaldehyd in methanolischem Medium umsetzt, zur Bildung der Dicarbonsäure der Formel (II):

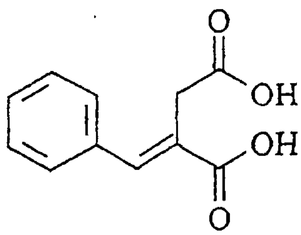

(II)

welche nach dem Erhitzen in aprotischem Medium in Gegenwart von Essigsäureanhydrid zu dem Anhydrid der Formel (III) führt:

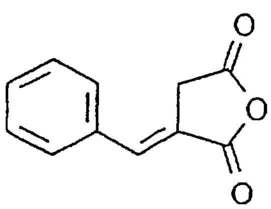

(III)

welches man mit Perhydroisoindolin der Formel (IV):

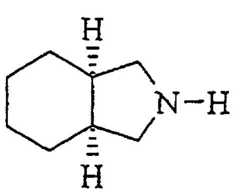

(IV)

in einem aprotischen Lösungsmittel umsetzt zur Bildung der Verbindung der Formel (V):

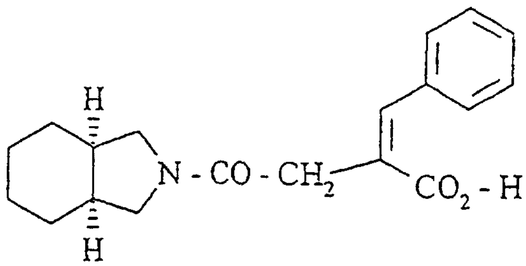

(V)

welches man einer katalytischen Hydrierung unterzieht unter Verwendung des Rhodium /(2S,4S)-N-Butoxycarbonyl-4-diphenylphosphino-2 -diphenylphosphinomethylpyrrolidin-Komplexes oder Rh/S,S) BPPM als asymmetrischem Hydrierkatalysator in Methanol oder Methylenchlorid als Medium, gefolgt von einer Salzbildung in Gegenwart eines Amins A zur Bildung der Verbindung der Formel (VI):

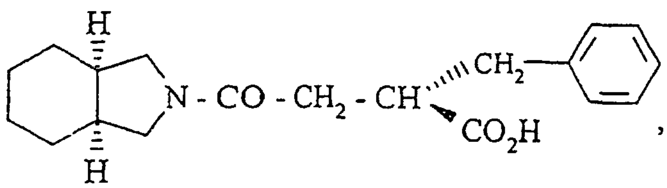

, A (VI)

welche:

in basischem Medium in Gegenwart eines anorganischen Salzes zu einem Additionssalz der Verbindung der Formel (I) führt, welche man gewünschtenfalls in die entsprechende Säure umwandelt,
oder in saurem Medium zu der Verbindung der Formel (I) führt, welche man gewünschtenfalls in ein pharmazeutisch annehmbares Additionssalz umwandelt.

**2.** Verfahren nach Anspruch 1, **dadurch gekennzeichnet, daß** das verwendete Amin A (R)-1-Phenylethylamin ist.

**3.** Verfahren nach Anspruch 1, **dadurch gekennzeichnet, daß** das Amin A Morpholin ist.

**4.** Verfahren nach Anspruch 1, **dadurch gekennzeichnet, daß** das Amin A N-Methylmorpholin ist.

**5.** Verfahren nach Anspruch 1, **dadurch gekennzeichnet, daß** das Amin A Cyclohexylamin ist.

**6.** Verfahren nach Anspruch 1 zur Herstellung des Calciumsalzes der Bis-2-(S)-benzyl-4-oxo-4-(cis-perhydroisoindol-2-yl)-buttersäure.

**7.** Verfahren nach Anspruch 1 zur Herstellung des Bis-2-(S)-benzyl-4-oxo-4-(cis-perhydroisoindol-2-yl)-buttersäure-calciumsalz-Dihydrats.